# EUROPEAN PATENT SPECIFICATION

(11) **EP 2 757 155 B1**
(45) Date of publication and mention of the grant of the patent: **29.06.2016**
(21) Application number: 12832210.4
(22) Date of filing: 11.09.2012
(51) Int. Cl.: C12N 15/09, A61K 39/04, A61P 31/06, C12N 1/21

(54) **RECOMBINANT BCG VACCINE**
REKOMBINANTER BCG-IMPFSTOFF
VACCIN RENFERMANT BCG RECOMBINANT

(30) Priority: 13.09.2011 JP 2011199422
(43) Date of publication of application: 23.07.2014
(73) Proprietor: Japan BCG Laboratory, Tokyo 112-0012 (JP); National Institutes of Biomedical Innovation, Health and Nutrition, Osaka 567-0085 (JP)
(72) Inventor: MATSUO, Kazuhiro, Kiyose-shi Tokyo 204-0022 (JP); MIZUNO, Satoru, Kiyose-shi Tokyo 204-0022 (JP); KAWAHARA, Mamoru, Kiyose-shi Tokyo 204-0022 (JP); YASUTOMI, Yasuhiro, Tsukuba-shi Ibaraki 305-0843 (JP); WATANABE, Kenta, Tsukuba-shi Ibaraki 305-0843 (JP)
(74) Representative: Boult Wade Tennant
(86) International application number: PCT/JP2012/073213
(87) International publication number: WO 2013/039069

(56) References cited:
- KATTI MURALIDHAR K ET AL: "The Delta fbpA mutant derived from Mycobacterium tuberculosis H37Rv has an enhanced susceptibility to intracellular antimicrobial oxidative mechanisms, undergoes limited phagosome maturation and activates macrophages and dendritic cells", CELLULAR MICROBIOLOGY, vol. 10, no. 6, June 2008 (2008-06), pages 1286-1303, XP002736377, ISSN: 1462-5814
- YEDDULA NARAYANA ET AL: "NOTCH1 Up-regulation and Signaling Involved in Mycobacterium bovis BCG-induced SOCS3 Expression in Macrophages", JOURNAL OF BIOLOGICAL CHEMISTRY, vol. 283, no. 18, 2 May 2008 (2008-05-02), pages 12501-12511, XP55170163, ISSN: 0021-9258, DOI: 10.1074/jbc.M709960200
- YASUKAWA H ET AL: "THE SUPPRESSOR OF CYTOKINE SIGNALING-1 (SOCS1) IS A NOVEL THERAPEUTIC TARGET FOR ENTEROVIRUS-INDUCED CARDIAC INJURY", JOURNAL OF CLINICAL INVESTIGATION, AMERICAN SOCIETY FOR CLINICAL INVESTIGATION, US, vol. 111, no. 4, 1 February 2003 (2003-02-01), pages 469-478, XP001184770, ISSN: 0021-9738, DOI: 10.1172/JCI200316491
- CAROW BERIT ET AL: "Silencing Suppressor of Cytokine Signaling-1 (SOCS1) in Macrophages Improves Mycobacterium tuberculosis Control in an Interferon-gamma (IFN-gamma)-dependent Manner", JOURNAL OF BIOLOGICAL CHEMISTRY, vol. 286, no. 30, July 2011 (2011-07), pages 26873-26887, XP002736378,
- KENTA WATANABE: "SOCS1 Antagonist o Kumikonda BCG o Mochiita Shinki Kekkaku Vaccine no Kaihatsu", INTERNET CITATION, 7 June 2012 (2012-06-07), XP008173056, Retrieved from the Internet: URL:http://kaken.nii.ac.jp/d/p/22890255/20 10/3/ja.ja.html [retrieved on 2014-10-31]
- WANGOO, A. ET AL.: 'Bacille Calmette-Gue'rin (BCG)-associated inflammation and fibrosis: modulation by recombinant BCG expressing interferon-gamma (IFN-gamma)' CLIN. EXP. IMMUNOL. vol. 119, no. 1, 2000, pages 92 - 98, XP055148587
- LUO, Y. ET AL.: 'Recombinant Mycobacterium bovis bacillus Calmette-Gue'rin (BCG) expressing mouse IL-18 augments Th1 immunity and macrophage cytotoxicity' CLIN. EXP. IMMUNOL. vol. 137, no. 1, 2004, pages 24 - 34, XP055148592
- IMAI, KENICHI ET AL.: 'Mycobacterium bovis bacillus Calmette-Gue'rin infection promotes SOCS induction and inhibits IFN-gamma-stimulated JAK/STAT signaling in J774 macrophages' FEMS IMMUNOL. MED. MICROBIOL. vol. 39, no. 2, 2003, pages 173 - 180, XP055148598
- HANADA, TOSHIKATSU ET AL.: 'A mutant form of JAB/SOCS1 augments the cytokine-induced JAK/ STAT pathway by accelerating degradation of wild-type JAB/CIS family proteins through the SOCS-box' J. BIOL. CHEM. vol. 276, no. 44, 2001, pages 40746 - 40754, XP001184769
- SPRATT, JOANNE M. ET AL.: 'Identification of strong promoter elements of Mycobacterium smegmatis and their utility for foreign gene expression in mycobacteria' FEMS MICROBIOL. LETT. vol. 224, 2003, pages 139 - 142, XP055148610
- LIM, ENG MONG ET AL.: 'Recombinant Mycobacterium bovis BCG producing the N-terminal half of SIVmac251 Env antigen induces neutralizing antibodies and cytotoxic T lymphocyte responses in mice and guinea pigs' AIDS RES. HUM. RETROVIRUSES vol. 13, no. 18, 1997, pages 1573 - 1581, XP008173070
- ME'DERLE', I. ET AL.: 'Plasmidic versus insertional cloning of heterologous genes in Mycobacterium bovis BCG: impact on in vivo antigen persistence and immune responses' INFECT. IMMUN. vol. 70, no. 1, 2002, pages 303 - 314, XP055148616
- MATSUO, KAZUHIRO ET AL.: 'Cloning and expression of the gene for the cross-reactive alpha antigen of Mycobacterium kansasii' INFECT. IMMUN. vol. 58, no. 2, 1990, pages 550 - 556, XP002299042
- MATSUMOTO, SOHKICHI ET AL.: 'A stable Escherichia coli-mycobacteria shuttle vector 'pS0246' in Mycobacterium bovis BCG' FEMS MICROBIOL. LETT. vol. 135, 1996, pages 237 - 243, XP055148618
- SATORU MIZUNO ET AL.: 'Shinki Kekkaku Vaccine to shite no rBCG-SOCSldn no Sakusei to Hyoka' BULLETIN OF THE JAPANESE SOCIETY OF TUBERCULOSIS vol. 87, no. 3, 15 March 2012, page 281, XP008173047
- KENTA WATANABE: 'SOCS1 Antagonist o Kumikonda BCG o Mochiita Shinki Kekkaku Vaccine no Kaihatsu' 2010 NENDO KENKYU JISSEKI HOKOKUSHO 07 June 2012, XP008173056 Retrieved from the Internet: <URL:http://kaken.nii.ac.jp/d/p/22890255/20 10/3/ja.ja.html> [retrieved on 2012-10-12]
- FREY, KENNETH G. ET AL.: 'HSV-1-induced SOCS-1 expression in keratinocytes: use of a SOCS-1 antagonist to block a novel mechanism of viral immune evasion' J. IMMUNOL. vol. 183, no. 2, 2009, pages 1253 - 1262, XP055148620
- ZHOU, HONGSHENG ET AL.: 'Induction of CML28- specific cytotoxic T cell responses using co-transfected dendritic cells with CML28 DNA vaccine and SOCS1 small interfering RNA expression vector' BIOCHEM. BIOPHYS. RES. COMMUN. vol. 347, 2006, pages 200 - 207, XP024925487
- SHEN, LEI ET AL.: 'Silencing of SOCS1 enhances antigen presentation by dendritic cells and antigen-specific anti-tumor immunity' NAT. BIOTECHNOL. vol. 22, no. 12, 2004, pages 1546 - 1553, XP002561700

## Description

### Technical Field

The present invention relates to the production of a recombinant BCG expressing a dominant negative mutant of a suppressor of cytokine signaling and a vaccine for tuberculosis prevention containing the recombinant BCG.

### Background Art

A BCG vaccine (Mycobacterium bovis BCG), which is currently used in the world as a tuberculosis vaccine, exhibits an 80% protective effect on pediatric tuberculous meningitis and miliary tuberculosis, whereas it is not fully effective in preventing adult pulmonary tuberculosis. Tuberculosis is one of the world's three major infectious diseases, and 9.3 million new patients develop the disease each year in the world. In Japan, 25,000 or more new patients develop the disease, and Japan is classified as a country where tuberculosis is intermediately spread. Although there is an established therapy with a combination of four effective drugs, infection with multidrug resistant Mycobacterium tuberculosis, which is resistant to two or more of these drugs, has become prevalent in recent years, and moreover, there are many reports of the emergence of highly multidrug resistant bacteria, which are resistant to three or more of these drugs. In view of the foregoing, the development of an effective novel tuberculosis vaccine is an urgent task.

Currently, progress has been made in the molecular biological research of acid-fast bacteria including BCG, and development of a more effective tuberculosis vaccine achieved by modifying the biological properties of BCG through introduction of exogenous genes thereinto has been tried around the world (Non Patent Literatures 1 and 2, Patent Literatures 1 and 2). For example, overexpression of protective antigen molecules of Mycobacterium tuberculosis in BCG, incorporation of cytokine genes or the like into BCG, etc. have been tried, chiefly by AERAS Global TB Vaccine Foundation, which is an NPO in USA. Nevertheless, no sufficient preventive effects on tuberculosis have been achieved so far, and there is still no decisive breakthrough in overcoming this situation. Further, also in Japan, National Hospital Organization Kinki-Chuo Chest Medical Center has reported the preventive effect on tuberculosis using recombinant BCG into which a gene of, for example, α antigen Ag85B is incorporated (Non Patent Literature 3). Nevertheless, it seems that no sufficient effect has yet been achieved.

Cellular Microbiology, 2008, 10(6), 1286-1303 describes an antigen 85A deficient mutant of M. tuberculosis H37Rv which blocks the expression of SOCS-1 in macrophages and constitutes a vaccine candidate.

### Citation List

### Patent Literature

Patent Literature 1: JP- 2008-517013 A
Patent Literature 2: JP-2001-518781 A

### Non Patent Literature

Non Patent Literature 1: J. Immunology, 2007, 179: 8418 to 8424
Non Patent Literature 2: Tuberculosis 2009, Jan; 89 (1): 62 to 67 (2008)
Non Patent Literature 3: Antibiotics & Chemotherapy, Vol. 23, No.3 447 to 453 (2007)

### Summary of the Invention

### Problem to be solved by the invention

An object of the present invention is to provide a recombinant BCG capable of enhancing the production of various cytokines at the cytokine signal transduction level. Another object of the present invention is to provide a highly effective vaccine for tuberculosis prevention comprising the above-mentioned recombinant BCG as the active ingredient.

### Solution to Problem

To make a new attempt to enhance the efficacy of BCG as a tuberculosis vaccine, the present inventors focused on suppressor of cytokine signaling (SOCS), which negatively regulates the immune response of the host against acid-fast bacteria such as BCG. That is, SOCS molecules are known to regulate the production of various cytokines by suppressing the JAK/STAT signaling pathway (for example, Yoshimura et al., Nat Rev Immunol. 7, 454 to 65 (2007)). For example, there is a recent report that increased expression of SOCS in macrophages (M Φ), dendritic cells (DC), and the like due to infection with BCG resulted in the suppression of immune responses (Imai et al. FEMS Immunol. Med. Microbiol. 39, 173 to 180 (2003) and Narayana and Balaji J. Biol. Chem. 283, 12501 to 12511 (2008)).

There is suggestion of possibility that such increased expression of SOCS may limit the effect of a BCG vaccine. It is presumed that if the above-mentioned suppression of immune responses induced by the action of SOCS1 can be canceled, the effect of a BCG vaccine can be enhanced. SOCS forms an SOCS family consisting of eight molecular groups, among which SOCS1 has now been revealed to control the signal transduction of IFNγ, IFNα, IL-2, IL-4, IL-6, IL-7, IL-10, IL-12, IL-15 and IL-21.

In light of the above, the present inventors studied the production of a new recombinant BCG that suppresses the action of the SOCS1 molecule, thereby promoting the Th1-type immune response. First of all, they produced an SOCS1 dominant negative mutant (single amino acid change) (SOCS1dn), which is an antagonist that competitively inhibits the SOCS1 function, and then produced a recombinant BCG (rBCG-SOCS1dn) into which an expression vector of the above SOCS1dn is incorporated. An in vitro analysis revealed that cancellation of the suppression of JAK2 phosphorylation in cells infected with the above rBCG-SOCS1dn was observed, and suppression of phosphorylation by SOCS1 was successfully canceled.

A mouse inhalation infection experiment using the aforementioned recombinant BCG (rBCG-SOCS1dn) revealed enhanced Mycobacterium tuberculosis-specific immune responses, and revealed that the function of SOCS1 in infected DC was suppressed by the action of rBCG-SOCS1dn, resulting in enhanced acid-fast bacteria-specific immune responses. The present inventors completed the present invention based on the foregoing findings. It should be noted that the findings of the present invention are based on a concept that is totally different from the strategy reported in the aforementioned Non Patent Literatures 1, 2, and 3 and Patent Literatures 1 and 2, which is to aim at enhancing the activity of a BCG vaccine by introducing an antigen gene that functions in protecting against Mycobacterium tuberculosis infection into a BCG. To the best of the inventors' knowledge, a concept underlying the present invention, which is to try to develop a more effective vaccine achieved by controlling the signal transduction pathway in the host through incorporation of a host-derived protein gene into a BCG, has never been reported previously.

Exemplary embodiments provided by the present invention are given in (1) to (20) below.
(1) An expression vector of a suppressor of cytokine signaling for recombination of a BCG, comprising:
   a) a DNA consisting of a nucleotide sequence encoding a dominant negative mutant of suppressor of cytokine signaling 1 (SOCS1) having a single amino acid substitution in the amino acid sequence of SOCS1 or a DNA consisting of a nucleotide sequence having 90% or more identity to the nucleotide sequence encoding SOCS1 and containing the single amino acid substitution, and
   b) a DNA consisting of a nucleotide sequence encoding a signal peptide.
(2) An expression vector of a suppressor of cytokine signaling for recombination of a BCG, comprising:
   a) a DNA consisting of a nucleotide sequence encoding a dominant negative mutant of suppressor of cytokine signaling 1 (SOCS1) having a single amino acid substitution in the amino acid sequence of SOCS1,
   b) a DNA consisting of a nucleotide sequence encoding a transcription promoter of a gene selected from the group consisting of SP2, hsp60, hsp70 and Antigen 85B,
   c) a DNA consisting of a nucleotide sequence encoding a signal peptide of a gene selected from the group consisting of blaF, blaC, Antigen 85B and MPB64,
   d) a DNA consisting of an SD sequence, and
   e) a DNA consisting of a nucleotide sequence encoding a transcription terminator of a gene selected from the group consisting of hsp60, Antigen 85B and an M. kansasii-derived α antigen.
(3) The expression vector according to (1) or (2), further comprising a kanamycin resistance gene.
(4) The expression vector according to any of (1) to (3), wherein the expression vector is an Escherichia coli-acid-fast bacteria shuttle vector, comprising:
   a) a DNA consisting of a nucleotide sequence encoding a dominant negative mutant of suppressor of cytokine signaling 1 (SOCS1) having a single amino acid substitution in the amino acid sequence of SOCS 1,
   b) a DNA consisting of a nucleotide sequence encoding a transcription promoter of a gene selected from the group consisting of SP2, hsp60, hsp70 and Antigen 85B,
   c) a DNA consisting of a nucleotide sequence encoding a signal peptide of a gene selected from the group consisting of blaF, blaC, Antigen 85B and MPB64,
   d) a DNA consisting of an SD sequence, and
   e) a DNA consisting of a nucleotide sequence encoding a transcription terminator of a gene selected from the group consisting of hsp60, Antigen 85B and an M. kansasii-derived α antigen.
(5) The expression vector according to (4), wherein the Escherichia coli-acid-fast bacteria shuttle vector is pSO246.
(6) The expression vector according to any of (1) to (5), wherein the nucleotide sequence encoding a signal peptide is a blaF signal sequence.
(7) The expression vector according to any of (1) to (6), wherein the nucleotide sequence encoding a transcription terminator is an Antigen 85B terminator sequence.
(8) The expression vector according to any of (1) to (7), wherein a site of the single amino acid substitution is located in a kinase inhibitory region of SOCS1.
(9) The expression vector according to any of (1) to (8), wherein a site of the single amino acid substitution is position 59 from an N-terminus.
(10) The expression vector according to any of (1) to (9), wherein an amino acid at position 59 is changed from phenylalanine to aspartic acid.
(11) The expression vector according to any of (1) to (10), wherein the nucleotide sequence encoding a dominant negative mutant of suppressor of cytokine signaling 1 (SOCS1) having a single amino acid substitution in the amino acid sequence of SOCS1 is SEQ ID NO: 1.
(12) A recombinant BCG (rBCG-SOCS1dn), which is obtained by introducing the expression vector according to any of (1) to (11) into a BCG.
(13) The recombinant BCG according to (12), wherein the BCG is a BCG Tokyo strain.
(14) A BCG vaccine, comprising the recombinant BCG according to (12) or (13).
(15) The BCG vaccine according to (14), wherein the BCG vaccine is a vaccine for tuberculosis prevention.
(16) The vaccine according to (14) or (15), wherein the vaccine enhances a capacity to induce cytokine and/or chemokine production.
(17) The vaccine according to any of (14) to (16), wherein the capacity to induce cytokine and/or chemokine production is a capacity to induce the production of at least one cytokine selected from the group consisting of IFN-γ, TNF-α and RANTES and/or a capacity to induce Antigen 85B-specific cell-mediated immunity.
(18) A method for protecting against tuberculosis infection, comprising administering the vaccine according to any of (14) to (17).
(19) A method for producing a BCG vaccine, comprising introducing the expression vector according to any of (1) to (11) into a BCG.
(20) A method for enhancing immunity against a BCG, comprising expressing in a BCG, a dominant negative mutant of suppressor of cytokine signaling 1 (SOCS1) having a single amino acid substitution in the amino acid sequence of SOCS1.

### Effects of the Invention

The present invention provides a recombinant BCG (rBCG-SOCS1dn) that is readily taken up by macrophages and dendritic cells when administered to the living body and produce a SOCS1 dn protein within the cells. The produced SOCS1 dn protein antagonistically acts against the SOCS1 protein which is intrinsically present in the cells, and thereby suppresses the inhibitory activity of SOCS1 on JAK phosphorylation. In one embodiment of the present invention, IFN-γ production capacity of the recombinant BCG is improved by enhancing IFN-γ signaling in an immunized individual, and the recombinant BCG exerts a strikingly higher immunity inducing capacity than the host parental BCG strain. Thus, a vaccine containing the above recombinant BCG as the active ingredient has an enhanced cytokine production capacity in the host and an enhanced immunity inducing capacity, both of which has not been reported for a tuberculosis vaccine.

### Brief Description of the Drawings

[Figure 1]

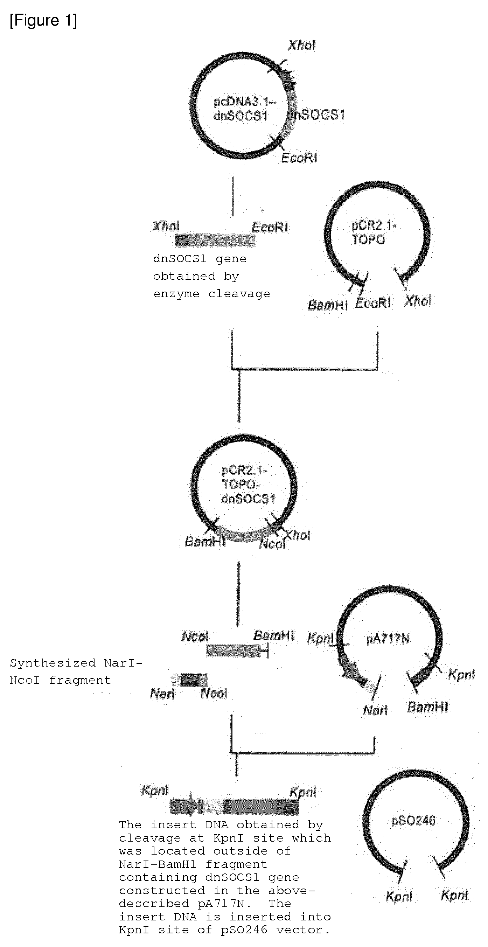

   Figure 1 is a flow chart showing a strategy for constructing an expression vector which expresses SOCS1dn gene (SEQ ID No.:1) in BCG.
[Figure 2]
   (a) The figure is a chart showing a structure of SOCS1dn expression vector which was finally constructed. In the figure, KmR indicates kanamycin resistance gene; SP2 indicates SP2 promoter, megaSD indicates Shine-Dalgarno (SD)sequence; blaF indicates signal peptide sequence of beta-lactamase gene derived from Mycobacterium fortuitum, and terminator indicates terminator of alpha-antigen derived from M. Kansasii.
   (b) The figure shows western blotting analysis where extract of the expression plasmid-introduced rBCG-SOCS1dn bacteria cell was subjected to SDS-polyacrylamide gel electrophoresis followed by blotting using PVDF membrane and a protein which reacts with anti-SOCS antibody was detected. A specific band is found at 37kDa.

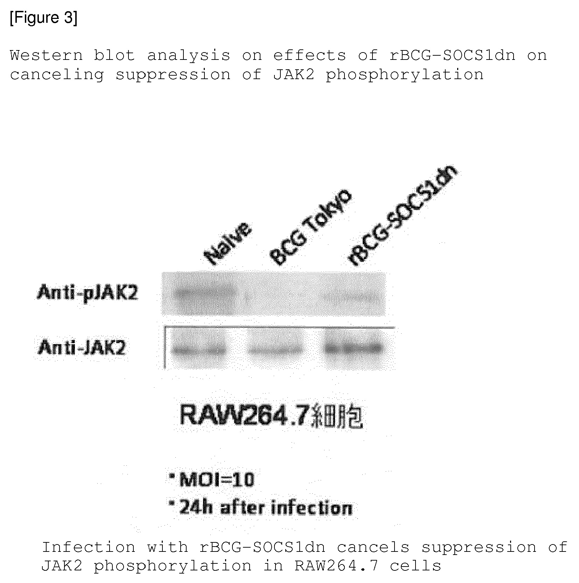

[Figure 3] The figure shows western blotting analysis where RAW264.7 cells were infected with BCG Tokyo strain and rBCG-SOCS1dn in vitro and phosphorylation of JAK2 protein was investigated.
[Figure 4]
   (a) The figure shows the number of cells producing IFN-gamma evaluated by the ELISPOT method after rBCG-SOCS1dn-immunized mouse spleen cells were stimulated by Antigen 85B protein or recombinant vaccinia virus expressing antigen 85B gene.
   (b) The figure shows results of determining production of TNF-alpha and RANTES in the culture supernatant after rBCG-SOCS1dn-immunized mouse spleen cells were stimulated by Antigen 85B protein or recombinant vaccinia virus expressing antigen 85B gene or stimulated by PPD.

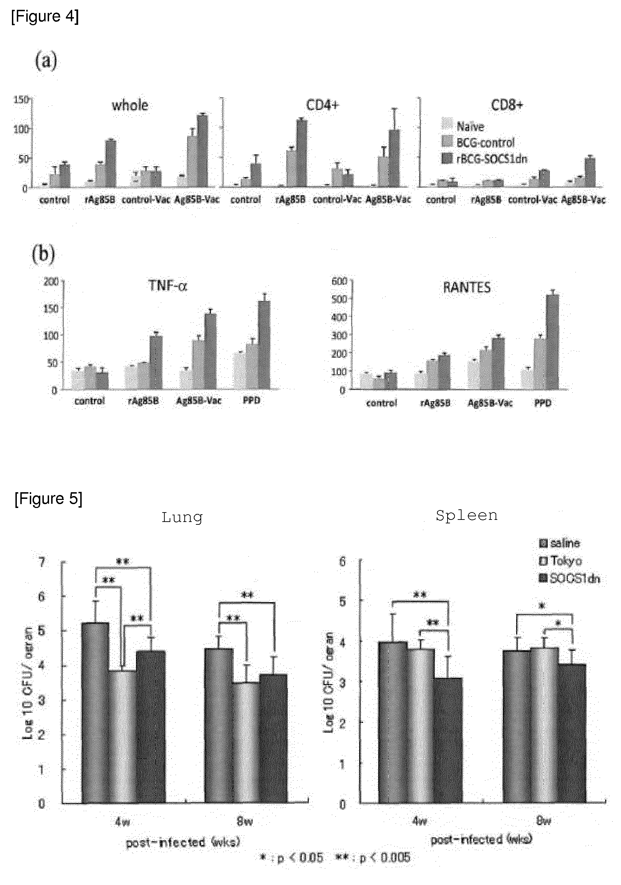

[Figure 5] The figure is a graph showing a comparison of the number of tubercle bacilli, which was determined by sub-culturing tubercle bacilli derived from lungs and spleen of rBCG-SOCSIdn-immunized mice or BCG Tokyo strain-immunized mice 4 or 8 weeks after infection with tubercle bacilli by inhalation.

### Description of Embodiments

### - First aspect of the present invention -

According to the first aspect of the present invention, a vector comprising a gene sequence of a SOCS1 dominant negative mutant is provided. Examples of the vector include a vector having the nucleotide sequence represented by SEQ ID NO: 2.

The "SOCS1 dominant negative mutant (SOCS1dn)" used in the present invention is a mutant protein that inhibits the SOCS1 activity, which can be a mutant protein resulting from a single amino acid substitution in the amino acids present in the kinase inhibitory region of the wild-type SOCS1 protein. Particularly preferable examples thereof include a protein resulting from the single amino acid substitution of Asp-59 for Phe-59 in the wild-type SOCS1 protein. A gene encoding such SOCS1dn can be obtained by introducing such a gene mutation that causes the single amino acid substitution of Asp-59 for Phe-59 encoded by the wild-type SOCS1 gene (Hanada et al. J. Biol. Chem. 276, 40746 to 40754 (2001)). Regarding a method for introducing such a mutation, publicly known methods such as a method including a method where a mutation is introduced in a PCR primer, followed by cloning PCR-amplified DNA containing the mutation, and site-specific mutagenesis according to the Kunkel's method can be used.

Also, the expression vector provided by the present invention may comprise a DNA encoding a SOCS1 mutant consisting of an amino acid sequence having 80% or more, 90% or more, 95% or more, 98% or more, or 99% or more identity to the amino acid sequence of wild-type SOCS1. A SOCS1 mutant consisting of the amino acid sequence having such identity can be a mutant protein that inhibits the SOCS1 activity, which results from a single amino acid substitution in the amino acids present in the kinase inhibitory region of the wild-type SOCS1 protein.

Examples of the SOCS1dn gene include a gene consisting of the nucleotide sequence represented by SEQ ID NO: 1.

In the present specification, the "expression vector" refers to a vector used for expressing a specific exogenous gene. Examples of the expression vector provided by the present invention include a plasmid DNA having a transcription promoter, a Shine-Dalgarno (SD) sequence, a signal peptide and a transcription terminator, which enables the secretion and expression of SOCS1dn in BCG, and a replicon that can be replicated in acid-fast bacteria such as BCG.

The "transcription promoter" used in the present invention is an important factor involved in the expression of the gene of interest of the present invention, and it can be one that is potent and capable of producing a large amount of mRNA. Preferable examples thereof include a promoter of, for example, SP2 (Spratt et al., FEMS Microbiol. Lett. 224: 139 to 142 (2003)), hsp60, hsp70 and Antigen 85B. More preferable examples include SP2.

The "transcription terminator" used in the present invention can be one that is incorporated for the purpose of terminating transcription and improving the turnover of RNA polymerase, and examples thereof include the terminator of, for example, hsp60, Antigen 85B and the M. kansasii-derived α antigen. Preferable examples include the terminator of Antigen 85B.

The "SD sequence" used in the present invention can be an important sequence to allow mRNA produced by transcription to bind to 3'-terminus of 16S ribosomal RNA due to the complementary sequence and initiate translation into protein. For example, along with a normal 4 to 5-base-long SD sequence, an 11-base-long SD sequence (AGAAGGAGAAG) called mega SD with a stronger binding force can be used (Mederie et al. Infect. Immun. 70: 303 to 314 (2002)). Preferable examples of the SD sequence include mega SD sequence.

The "signal peptide" used in the present invention can be a peptide sequence of 20 to 40 amino acid residues that are necessary for a secretory protein to pass across the membrane. Because SOCS1 is originally a protein that functions within the cytoplasm of the host cell and a protein functioning within the cytoplasm generally has a structure that makes the protein difficult to pass across the membrane, the SOCS1dn protein used in the present invention is also assumed to be difficult to pass across the cell membrane. In light of this, it was considered important to link SOCS1dn to a signal peptide which helps it to permeate cell membrane and to be secreted from the bacterial cell. Examples of the signal peptide used in the present invention include an acid-fast bacterium-derived signal peptide, which efficiently secretes the SOCS1dn protein from BCG. For example, a variety of acid-fast bacteria-derived signal peptide genes such as blaF (Timm et al. Mol. Microbiol. 12: 491 to 504 (1994)), blaC (McDonough et al. J. Bacteriol. 187: 7667 to 7679 (2005)), Antigen 85B (Matsuo et al. J. Bacteriol. 170: 3847 to 3854 (1988)) and MPB64 (Yamaguchi et al. Infect. Immun.57: 283 to 288 (1989)) can be used. Preferable examples include blaF.

Further, in the expression vector provided by the present invention, an antibiotic resistance gene, a replication origin (ori), and the like that are present in normal expression vectors may also be present. Examples of the antibiotic resistance gene include an ampicillin resistance gene, a tetracycline resistance gene, a kanamycin resistance gene (KmR), and a hygromycin resistance gene. The antibiotic resistance gene can be appropriately selected depending on the purpose. Examples of the replication origin include SV40, Col El ori, pUC ori, 2µ ori, pBR322 ori and Ori-M. The replication origin can be appropriately selected depending on the purpose.

The "Escherichia coli-acid-fast bacteria shuttle vector" used in the present invention can be a vector that can be expressed in both acid-fast bacteria such as BCG and Escherichia coli. The SOCS1dn protein can be further efficiently expressed by incorporating the SOCS1dn gene, a transcription promoter, a Shine-Dalgarno (SD) sequence, a signal peptide, and a transcription terminator in this vector. The Escherichia coli-acid-fast bacteria shuttle vector that can be used in the present invention is not particularly limited and can be a publicly known shuttle vector, and examples thereof include pSO246 and PNN2.

### - Second aspect of the present invention -

According to the second aspect of the present invention, a recombinant BCG transformed with a vector containing a gene sequence of a dominant negative mutant of SOCS1, in which phenylalanine (Phe) is changed to aspartic acid (Asp) at position 59, are provided.

In the present specification, "BCG" is an abbreviation of Bacille de Calmette et Guerin, which was named after the producer's name, and is Mycobacterium bovis that lost its virulence in humans and maintains only antigenicity after being subcultured. Examples of BCG include the BCG Tokyo strain.

Regarding a method for introducing an expression vector into a BCG, a method for introducing an expression vector into acid-fast bacteria can be used. A method for introducing the expression vector provided by the present invention into a BCG by electroporation can also be used. Further, it is also possible to insert the gene site-specifically into the genomic DNA using homologous recombination.

The "recombinant BCG" provided by the present invention can be a BCG that are enabled to express an SOCS1dn protein by recombination using the expression vector provided by the present invention. For selection of the recombinant BCG, after introducing the expression vector of the present invention into a BCG, the BCG is cultured for approximately three weeks at 37°C on agar media prepared by adding a drug such as kanamycin and hygromycin to commercially available acid-fast bacterial culture agar media such as 7H10, and the resulting colonies are picked up, whereby the recombinant BCG can be selected.

Further, the expression of the SOCS1dn protein in the recombinant BCG can be confirmed as follows. First of all, a fraction of the recombinant BCG is collected and cultured in a liquid medium such as 7H9 medium and Sauton's medium for approximately two weeks at 37°C for bacterial propagation, and then the bacterial cells are disrupted by means of, for example, ultrasonic disruption to prepare a bacterial cell extract. Then, proteins in culture supernatants are concentrated by a general biochemical method such as ammonium sulfate precipitation and trichloroacetic acid precipitation to prepare a mixture of bacterial cell secretory proteins. Then, after fractionating the obtained sample using SDS-polyacrylamide gel electrophoresis, the proteins are electrically blotted onto a filter made of, for example, nitrocellulose. Subsequently, the expression of the SOCS1dn protein can be confirmed by Western blotting to detect proteins reacting with a commercially available anti-SOCS1 antibody.

### - Third aspect of the present invention -

According to the third aspect of the present invention, a BCG vaccine comprising the aforementioned recombinant BCG as an active ingredient is provided.

The "BCG vaccine" provided by the present invention can be a live BCG vaccine having an enhanced capacity to produce various cytokines such as IFN-γ. For example, the BCG vaccine provided by the present invention can be a vaccine having an enhanced capacity to induce the production of at least one cytokine selected from the group consisting of IFN-γ, THF-α and RANTES and/or an enhanced capacity to induce Antigen 85B-specific cell-mediated immunity. Preferably, the BCG vaccine provided by the present invention is used as a tuberculosis prevention vaccine.

In one embodiment, the present invention provides a BCG vaccine that not only increases the number of IFN-γ-producing cells, but also enhances the capacity to induce Antigen 85B-specific cell-mediated immunity to a greater degree, leading to a greater amount of TNF-α and RANTES production, compared to a vaccine derived from the host BCG Tokyo strain.

Also, in one embodiment, the BCG vaccine provided by the present invention induces increased production of cytokines and chemokines (for example, IFN-γ, THF-α, and RANTES) in a subject (human) due to the administered SOCS1 dominant negative mutant (SOCS1dn), thereby enhancing immunity against BCG (for example, antibody production against BCG).

The "capacity to induce cytokine and chemokine production" can serve as an index for evaluating the immunogenicity of the recombinant BCG provided by the present invention, which can be evaluated by the following method. First of all, a suspension of an appropriate number of live cells of the recombinant BCG in a medium such as physiological saline is subcutaneously inoculated into a small animal such as a mouse and a guinea pig. After two to eight weeks, spleen cells are collected and stimulated with BCG or Mycobacterium tuberculosis-derived antigenic proteins such as Antigen 85B and PPD, and the number of IFN-γ-producing cells is counted by the ELISPOT assay, whereby the immunogenicity of the recombinant BCG can be evaluated. Further, the cytokine or chemokine production capacity can be evaluated by culturing the spleen cells of the aforementioned sensitized animal for a certain period of time in vitro, and then determining the concentrations of various cytokines or chemokines in culture supernatants by the ELISA assay. As a result of the aforementioned evaluation tests, it was revealed that the BCG vaccine produced in Examples of the present specification not only increased the number of IFN-γ-producing cells, but also enhanced the capacity to induce Antigen 85B-specific cell-mediated immunity, leading to an increased amount of TNF-α and RANTES production in the animal (such as a mouse) receiving the administration of the BCG vaccine.

The BCG vaccine provided by the present invention can be administered to humans in the form of a similar formulation to the conventional BCG vaccine. The dosage can be appropriately adjusted in consideration of the effect.

Also, the BCG vaccine provided by the present invention can be a composition for the prevention of tuberculosis, which can be formulated into a drug with a pharmaceutically acceptable carrier (including an additive). Examples of the pharmaceutically acceptable carrier include, but are not limited to, an excipient (such as dextrin, hydroxypropylcellulose, and polyvinylpyrrolidone), a disintegrant (such as carboxymethylcellulose), a lubricant (such as magnesium stearate), a surfactant (such as sodium lauryl sulfate), a solvent (such as water, saline, and soybean oil) and a preservative (such as p-hydroxybenzoate). Thus, in one embodiment, the present invention provides a method for producing a BCG vaccine, comprising mixing the BCG provided by the second aspect of the present invention with a pharmaceutically acceptable carrier. Further, in one embodiment, the present invention provides a method for protecting against tuberculosis infection, comprising administering the BCG vaccine provided by the present invention to a human.

Hereinbelow, the present invention will be more specifically described based on Examples and Test Examples; however, the present invention is not limited by these Examples in any way.

### Examples

Hereinbelow, the present invention will be more specifically described based on Examples and Test Examples; however, the present invention is not limited by these Examples in any way.

### (Example 1) Construction of the SOCS1dn secretory expression vector

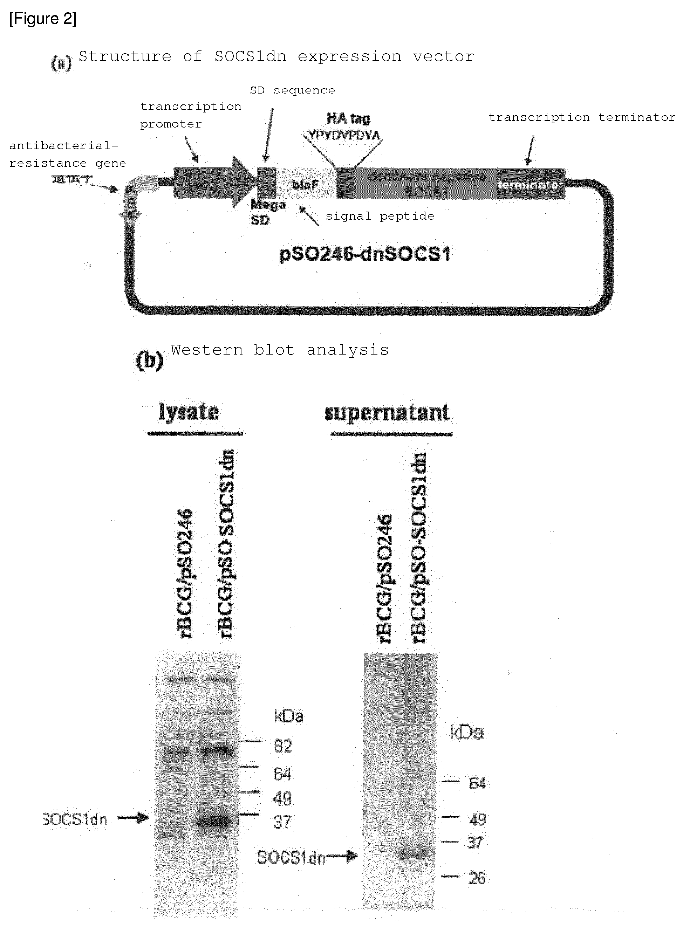

The construction flow chart is illustrated in Figure 1. First of all, a pA717N vector (SEQ ID NO: 3) was prepared by incorporating an expression cassette, in which an SP2 promoter (Spratt et al., FEMS Microbiol. Lett. 224: 139 to 142 (2003)), a megaSD sequence (Mederie et al. Infect. Immun. 70: 303 to 314 (2002)), a signal peptide gene of the M. fortuitum-derived β-lactamase gene (blaF) (Genbank L25634 (nucleotide Nos. 1274 to 1369)) and the M. kansasii-derived α antigen terminator gene (Genbank X53897 (nucleotide Nos. 1221 to 1396)) are arranged in this order from 5', into a pUC18 plasmid. Subsequently, the SOCS1dn gene having an HA tag at the N terminus, which is cloned in pcDNA3.1 (the product of Invitrogen), was cut out by XhoI-EcoRI digestion and subcloned in a pCR2.1-TOPO vector to obtain pCR2.1-TOPO-SOCS1dn. From this plasmid, a DNA fragment containing from Ncol site to the stop codon was cut out by NcoI-BamHI digestion and cloned in a pA717N plasmid at the Narl-BamHI site, which is located downstream of the SP2 promoter, via a synthetic DNA adapter containing a part of the 3'-terminus of the blaF signal sequence and a part of the 5'-terminus of SOCS1dn to obtain pA717N-SOCS1dn (SEQ ID NO: 2). From this plasmid, the SOCS1dn expression cassette was cut out by Kpnl digestion and introduced to the Kpnl site in the Escherichia coli-acid-fast bacteria shuttle vector, pSO246 [Matsumoto et al. FEMS Microbiol Lett. 135: 237 to 43 (1996)] to obtain the SOCS1dn secretory expression vector, pSO-SOCS1dn (Figure 2(a)).

### (Example 2) Construction of recombinant BCG expressing SOCS1dn

The BCG Tokyo strain was transformed with pSO-SOCS1dn and pSO246 (SEQ ID NO: 4) by electroporation in accordance with a conventional method and cultured on 7H10-OADC agar media (the product of Becton, Dickinson and Company) with kanamycin for two to three weeks at 37°C. Then, colonies were picked up and cultured in 7H9-ADC enrichment liquid media (the product of Becton, Dickinson and Company) with kanamycin for two weeks while shaking. When OD at 600 nm reached 0.5, the bacterial cells were collected by centrifugation and subjected to ultrasonic disruption to prepare a bacterial cell extract. A fraction of the resulting bacterial cell extract was subjected to SDS-PAGE and then blotted onto a PVDF membrane, which was reacted with a goat polyclonal anti-SOCS1 antibody (primary antibody) and with an HRP-labeled anti-goat IgG antibody (secondary antibody) (the product of GeneTex, Inc.) (Western blot analysis). For band detection, the ECL Advanced Western Blotting Detection Kit (the product of GE Healthcare) was used. The results are shown in Figure 2(b). BCG into which pSO-SOCS1dn was introduced showed a unique band that was not observed with BCG into which an empty pSO246 vector was introduced, showing the high-level expression of SOCS1dn proteins. Meanwhile, the same recombinant BCG clone was cultured in a 7H9 liquid medium without ADC enrichment (the product of Becton, Dickinson and Company), which contains 2% glycerol and kanamycin for two to three weeks at 37°C. When OD at 600 nm reached 0.5, the bacterial cells were removed by centrifugation to collect a supernatant, which was then passed through a 0.45 µm filter (the product of Millipore Corporation) for complete removal of bacteria. To 1 ml of the resulting supernatant, an equal volume of a 10% aqueous solution of trichloroacetic acid was added, followed by mixing. The resulting mixture was placed still on ice for 30 minutes and then subjected to centrifugation to collect a mixture of precipitated proteins. A similar Western blot analysis to the above was performed on this sample and revealed that the expressed SOCS1dn proteins were secreted out of the BCG cells.

### (Test Example 1) Analysis on effects of rBCG-SOCS1dn on canceling the suppression of JAK2 phosphorylation (in vitro assay)

The RAW246.7 cells were seeded in a 12 well plate at 2 x 10⁵ cells/well and cultured in an RPMI1640 medium at 37°C in the presence of 5% CO2 for 18 hours. Then, BCG-Tokyo or rBCG-SOCS1dn was added so as to achieve MOI = 10, followed by further culture for 24 hours. After completion of the culture, the cells were collected, to which 100 µl of a Lysis Buffer (50 mM Tris-HCl (pH 7.4), 250 mM NaCl, 50 mM NaF, 5 mM EDTA, 0.1% Nonidet P-40 and 1% protease inhibitor mix (the product of GE Healthcare) was added, followed by lysis at 4°C for one hour. Subsequently, the cells were disrupted by ultrasonic treatment to obtain a cell extract. After developing the resulting cell extract by SDS polyacrylamide gel electrophoresis, proteins were blotted to a PVDF membrane and analyzed by Western blot using a rabbit anti-JAK2 antibody (the product of EPITOMICS) and a rabbit anti-pJAK2 antibody (the product of EPITOMICS). The results are shown in Figure 3.

In BCG Tokyo-infected RAW264.7 cells, JAK2 phosphorylation was suppressed to a greater degree than non-infected cells, which is presumed to be the effect of overexpression of SOCS1. On the other hand, in rBCG-SOCS1dn-infected cells, JAK phosphorylation was observed, revealing that suppression of phosphorylation by SOCS1 was canceled.

### (Test Example 2) Test of enhanced production of BCG-specific IFN-γ by rBCG-SOCS1dn in mice

Balb/c mice were subcutaneously inoculated with the BCG Tokyo strain or rBCG-SOCS1dn, 0.1 mg each, and after four weeks, the spleen was harvested and spleen cells were prepared, which were stimulated with recombinant Antigen 85B protein or an Antigen 85B-expressing recombinant vaccinia virus. Subsequently, the number of IFN-γ-producing cells was counted by a commercially available ELISPOT kit (the product of MABTEC). The results are shown in Figure 4(a). In the rBCG-SOCS1dn immunization group, spot-forming cells were observed at a significantly higher frequency than the BCG Tokyo strain immunization group, revealing that the capacity to induce Antigen 85B-specific cell-mediated immunity was enhanced. Also, spleen cells of immunized mice were cultured after stimulation with various antigens, and TNF-α and RANTES in culture supernatants were quantified. As a result, the rBCG-SOCS1dn immunization group showed higher concentrations of TNF-α and RANTES than the BCG Tokyo immunization group, revealing that the capacity to induce cytokine and chemokine production was enhanced (Figure 4(b)).

### (Test Example 3) Test of enhanced protective capacity against Mycobacterium tuberculosis infection by rBCG-SOCS1dn in mice

Balb/c mice were subcutaneously inoculated with the BCG Tokyo strain and rBCG-SOCS1dn, 0.5 mg each. As a negative control, a group receiving subcutaneous inoculation of only physiological saline was prepared. Four weeks after immunization, the mice were infected with the Mycobacterium tuberculosis H37Rv strain (47 colony forming units per mouse) by aerosol infection using the aerosol infection device, model 099C A4212, manufactured by Glas-Col, LLC. Four and eight weeks after infection, the lungs and spleen were harvested and certain weights of these organs were ground by a grinder, and then seeded and cultured in 1% Ogawa's media (the product of Japan BCG Laboratory). After four weeks of culture, the number of colonies of Mycobacterium tuberculosis was counted, and the potentiating effect on protective capacity against infection was evaluated based on whether the resulting bacterial count was less than that obtained with the BCG Tokyo strain-immunized mice (Figure 5). With respect to the lungs, there was no difference between immunization with the BCG Tokyo strain and with rBCG-SOCS1dn. Rather, the parental BCG strain tended to exhibit a higher protective effect. However, with respect to the spleen, the rBCG-SOCS1dn-immunized mice showed a lower bacterial count, revealing enhanced protective effect against Mycobacterium tuberculosis.

### Industrial Applicability

A recombinant BCG produced by using a SOCS1dn protein expression vector that can be provided by the present invention can secrete the SOCS1dn protein out of the bacterial cells, and thereby can enhance the capacity to induce cytokine and chemokine production in macrophages and dendritic cells. Hence, a BCG vaccine containing the recombinant BCG vaccine that can be provided by the present invention as the active ingredient can be a tuberculosis vaccine having enhanced efficacy, and therefore can be a promising tuberculosis prevention vaccine against multidrug resistant Mycobacterium tuberculosis, the prevalence of which has been a concern in recent years.

## Claims

1. An expression vector of a suppressor of cytokine signaling for recombination of a BCG, **characterized in that** the expression vector is the Escherichia coli-acid-fast bacteria shuttle vector pSO246 comprising:
a) a DNA consisting of a nucleotide sequence encoding a dominant negative mutant of suppressor of cytokine signaling 1 (SOCS1) having a single amino acid substitution in the amino acid sequence of SOCS1, wherein the site of the single amino acid substitution is position 59 from the N-terminus and the amino acid is changed from phenylalanine to aspartic acid,
b) a DNA consisting of a nucleotide sequence encoding a transcription promoter of a gene selected from the group consisting of SP2, hsp60, hsp70 and Antigen 85B,
c) a DNA consisting of a nucleotide sequence encoding a signal peptide of a gene selected from the group consisting of blaF, blaC, Antigen 85B and MPB64,
d) a DNA consisting of an SD sequence, and
e) a DNA consisting of a nucleotide sequence encoding a transcription terminator of a gene selected from the group consisting of hsp60, Antigen 85B and an M. kansasii-derived α antigen.

2. The expression vector according to claim 1, further comprising a kanamycin resistance gene.

3. The expression vector according to claim 1 or claim 2, wherein the nucleotide sequence encoding a signal peptide is a blaF signal sequence.

4. The expression vector according to any of claims 1 to 3, wherein the nucleotide sequence encoding a transcription terminator is an Antigen 85B terminator sequence.

5. The expression vector according to any of claims 1 to 4, wherein the nucleotide sequence encoding a dominant negative mutant of suppressor of cytokine signaling 1 (SOCS1) is represented by SEQ ID NO: 1.

6. A recombinant BCG (rBCG-SOCS1dn) obtained by introducing the expression vector according to any of claims 1 to 5 into a BCG.

7. A BCG vaccine comprising the recombinant BCG according to claim 6.

8. The vaccine according to claim 7, wherein the vaccine enhances a capacity to induce cytokine and chemokine production.

9. A composition for use in preventing the development of tuberculosis, comprising the BCG according to claim 6.

## Patentansprüche

1. Expressionsvektor eines Suppressors des Zytokin-Signalings für die Rekombination eines BCG, **dadurch gekennzeichnet, dass** der Expressionsvektor der säurefeste Escherichia coli-Bakterien-Shuttle-Vektor pSO246 ist, umfassend:
a) eine DNA, bestehend aus einer Nukleotidsequenz, welche eine dominant-negative Mutante des Suppressors des Zytokin-Signalings 1 (SOCS1) mit einer einzelnen Aminosäuresubstitution in der Aminosäuresequenz von SOCS1 codiert, wobei die Stelle der einzelnen Aminosäuresubstitution Position 59 vom N-Terminus ist und die Aminosäure von Phenylalanin zu Asparaginsäure geändert wird,
b) eine DNA, bestehend aus einer Nukleotidsequenz, welche einen Transkriptionspromotor eines Gens, ausgewählt aus der Gruppe, bestehend aus SP2, hsp60, hsp70 und Antigen 85B, codiert,
c) eine DNA, bestehend aus einer Nukleotidsequenz, welche ein Signalpeptid eines Gens, ausgewählt aus der Gruppe, bestehend aus blaF, blaC, Antigen 85B und MPB64, codiert,
d) eine DNA, bestehend aus einer SD-Sequenz, und
e) eine DNA, bestehend aus einer Nukleotidsequenz, welche einen Transkriptionsterminator eines Gens, ausgewählt aus der Gruppe, bestehend aus hsp60, Antigen 85B und einem M. kansasii-abgeleiteten α-Antigen, codiert.

2. Expressionsvektor gemäß Anspruch 1, welcher weiterhin ein Kanamycin-Resistenzgen umfasst.

3. Expressionsvektor gemäß Anspruch 1 oder Anspruch 2, wobei die ein Signalpeptid codierende Nukleotidsequenz eine blaF-Signalsequenz ist.

4. Expressionsvektor gemäß mindestens einem der Ansprüche 1 bis 3, wobei die einen Transkriptionsterminator codierende Nukleotidsequenz eine Antigen 85B-Terminatorsequenz ist.

5. Expressionsvektor gemäß mindestens einem der Ansprüche 1 bis 4, wobei die Nukleotidsequenz, welche eine dominant-negative Mutante des Suppressors des Zytokin-Signalings 1 (SOCS1) codiert, durch SEQ ID NO: 1 dargestellt wird.

6. Rekombinanter BCG (rBCG-SOCS1dn), erhalten durch Einbringen des Expressionsvektors gemäß mindestens einem der Ansprüche 1 bis 5 in einen BCG.

7. BCG-Impfstoff, welcher den rekombinanten BCG gemäß Anspruch 6 umfasst.

8. Impfstoff gemäß Anspruch 7, wobei der Impfstoff eine Fähigkeit zur Induzierung von Cytokin- und Chemokin-Produktion verstärkt.

9. Zusammensetzung zur Verwendung bei der Prävention der Entwicklung von Tuberkulose, welche den BCG gemäß Anspruch 6 umfasst.

## Revendications

1. Vecteur d'expression d'un suppresseur de la signalisation des cytokines pour la recombinaison d'un BCG, **caractérisé en ce que** le vecteur d'expression est le vecteur navette de la bactérie acido-alcoolo-résistante Escherichia coli pS0246, comprenant :
a) un ADN constitué d'une séquence nucléotidique codant pour un mutant dominant négatif du suppresseur de la signalisation des cytokines 1 (SOCS1) présentant une substitution d'acide aminé unique dans la séquence d'acides aminés de SOCS1, où le site de la substitution d'acide aminé unique est en position 59 à partir de l'extrémité N-terminale et l'acide aminé est changé de phénylalanine en acide aspartique,
b) un ADN constitué d'une séquence nucléotidique codant pour un promoteur de la transcription d'un gène choisi dans le groupe constitué de SP2, hsp60, hsp70 et Antigène 85B,
c) un ADN constitué d'une séquence nucléotidique codant pour un peptide signal d'un gène choisi dans le groupe constitué de blaF, blaC, Antigène 85B et MPB64,
d) un ADN constitué d'une séquence SD, et
e) un ADN constitué d'une séquence nucléotidique codant pour un terminateur de la transcription d'un gène choisi dans le groupe constitué de hsp60, Antigène 85B et un antigène α dérivé de M. kansasii.

2. Vecteur d'expression selon la revendication 1, comprenant en outre un gène de résistance à la kanamycine.

3. Vecteur d'expression selon la revendication 1 ou la revendication 2, dans lequel la séquence nucléotidique codant pour un peptide signal est une séquence signal de blaF.

4. Vecteur d'expression selon l'une quelconque des revendications 1 à 3, dans lequel la séquence nucléotidique codant pour un terminateur de la transcription est une séquence terminateur de l'Antigène 85B.

5. Vecteur d'expression selon l'une quelconque des revendications 1 à 4, dans lequel la séquence nucléotidique codant pour un mutant dominant négatif du suppresseur de la signalisation des cytokines 1 (SOCS1) est représentée par SEQ ID NO : 1.

6. BCG recombinant (rBCG-SOCS1dn) obtenu par l'introduction du vecteur d'expression selon l'une quelconque des revendications 1 à 5 dans un BCG.

7. Vaccin BCG comprenant le BCG recombinant selon la revendication 6.

8. Vaccin selon la revendication 7, le vaccin amplifiant la capacité d'induire la production des cytokines et des chimiokines.

9. Composition pour une utilisation dans la prévention du développement de la tuberculose, comprenant le BCG selon la revendication 6.


### SEQUENCE LISTING

<110> JAPAN BCG LABORATORY
   National Institute of Biomedical Innovation
<120> Novel recombinant BCG vaccine
<130> 671060
<150> JP 2011-199422
   <151> 2011-09-13
<160> 4
<170> PatentIn version 3.2
<210> 1
   <211> 639
   <212> DNA
   <213> Artificial
<220>
   <223> SOCS1dn
<400> 1
   atggtagcac gcaaccaggt ggcagccgac aatgcgatct ccccggcagc agagccccga      60
   cggcggtcag agccctcctc gtcctcgtct tcgtcctcgc cagcggcccc cgtgcgtccc     120
   cggccctgcc cggcggtccc agccccagcc cctggcgaca ctcacttccg caccgaccgc     180
   tcccactccg attaccggcg catcacgcgg accagcgcgc tcctggacgc ctgcggcttc     240
   tattggggac ccctgagcgt gcacggggcg cacgagcggc tgcgtgccga gcccgtgggc     300
   accttcttgg tgcgcgacag tcgccaacgg aactgcttct tcgcgctcag cgtgaagatg     360
   gcttcgggcc ccacgagcat ccgcgtgcac ttccaggccg gccgcttcca cttggacggc     420
   agccgcgaga ccttcgactg ccttttcgag ctgctggagc actacgtggc ggcgccgcgc     480
   cgcatgttgg gggccccgct gcgccagcgc cgcgtgcggc cgctgcagga gctgtgtcgc     540
   cagcgcatcg tggccgccgt gggtcgcgag aacctggcgc gcatccctct taacccggta     600
   ctccgtgact acctgagttc cttccccttc cagatctga                            639
<210> 2
   <211> 3847
   <212> DNA
   <213> Artificial
<220>
   <223> pA717N-HAtag-SOCS1dn
<400> 2
   tcgcgcgttt cggtgatgac ggtgaaaacc tctgacacat gcagctcccg gagacggtca      60
   cagcttgtct gtaagcggat gccgggagca gacaagcccg tcagggcgcg tcagcgggtg     120
   ttggcgggtg tcggggctgg cttaactatg cggcatcaga gcagattgta ctgagagtgc     180
   accatatatg cggtgtgaaa taccgcacag atgcgtaagg agaaaatacc gcatcaggcg    240
   cgccattcgc cattcaggct gcgcaactgt tgggaagggc gatcggtgcg ggcctcttcg    300
   ctattacgcc agctggcgaa agggggatgt gctgcaaggc gattaagttg ggtaacgcca    360
   gggttttccc agtcacgacg ttgtaaaacg acggccagtg ccaagcttgc atgcctgcag    420
   aattcgagct cggtaccatg caggtggcgc gccgcggcct caccaacgcg attgtgagct    480
   atctcgagga caacgggtca gggcgacgtg agtaaccagt aaccgggttg tgacctccct    540
   tggtagcggc atttccgcta ccaacaagta ggctgttgca tatccccgtg tgtacgacca    600
   gcacggcata ctctagaagg agaagtacat atgaccggac tatcgcgacg caacgttctg    660
   atcggttcgc tcgtggcggc agctgccgtc ggtgcgggcg tcggtggcgc cgcaccggca    720
   ttcgccgtgt acccctacga cgtgcccgac tacgccatgg tagcacgcaa ccaggtggca    780
   gccgacaatg cgatctcccc ggcagcagag ccccgacggc ggtcagagcc ctcctcgtcc    840
   tcgtcttcgt cctcgccagc ggcccccgtg cgtccccggc cctgcccggc ggtcccagcc    900
   ccagcccctg gcgacactca cttccgcacc gaccgctccc actccgatta ccggcgcatc    960
   acgcggacca gcgcgctcct ggacgcctgc ggcttctatt ggggacccct gagcgtgcac   1020
   ggggcgcacg agcggctgcg tgccgagccc gtgggcacct tcttggtgcg cgacagtcgc   1080
   caacggaact gcttcttcgc gctcagcgtg aagatggctt cgggccccac gagcatccgc   1140
   gtgcacttcc aggccggccg cttccacttg gacggcagcc gcgagacctt cgactgcctt   1200
   ttcgagctgc tggagcacta cgtggcggcg ccgcgccgca tgttgggggc cccgctgcgc   1260
   cagcgccgcg tgcggccgct gcaggagctg tgtcgccagc gcatcgtggc cgccgtgggt   1320
   cgcgagaacc tggcgcgcat ccctcttaac ccggtactcc gtgactacct gagttccttc   1380
   cccttccaga tctgagaatt ccagcacact ggcggccgtt actagtggat cctcgcgcaa   1440
   cggttgccgc tactgggctt gacggcaaga cgccgtacag tagtgtgttc ggcaccttga   1500
   acgctggtcc gccatgttca acgagccggt ctacctgccc gcaccgaaca agctggtgac   1560
   atcgacccat gcggtgcggt accgagctcg aattaccggt aagcttctgc agaattcgta   1620
   atcatgtcat agctgtttcc tgtgtgaaat tgttatccgc tcacaattcc acacaacata   1680
   cgagccggaa gcataaagtg taaagcctgg ggtgcctaat gagtgagcta actcacatta   1740
   attgcgttgc gctcactgcc cgctttccag tcgggaaacc tgtcgtgcca gctgcattaa   1800
   tgaatcggcc aacgcgcggg gagaggcggt ttgcgtattg ggcgctcttc cgcttcctcg   1860
   ctcactgact cgctgcgctc ggtcgttcgg ctgcggcgag cggtatcagc tcactcaaag   1920
   gcggtaatac ggttatccac agaatcaggg gataacgcag gaaagaacat gtgagcaaaa   1980
   ggccagcaaa aggccaggaa ccgtaaaaag gccgcgttgc tggcgttttt ccataggctc   2040
   cgcccccctg acgagcatca caaaaatcga cgctcaagtc agaggtggcg aaacccgaca   2100
   ggactataaa gataccaggc gtttccccct ggaagctccc tcgtgcgctc tcctgttccg 2160
   accctgccgc ttaccggata cctgtccgcc tttctccctt cgggaagcgt ggcgctttct 2220
   catagctcac gctgtaggta tctcagttcg gtgtaggtcg ttcgctccaa gctgggctgt 2280
   gtgcacgaac cccccgttca gcccgaccgc tgcgccttat ccggtaacta tcgtcttgag 2340
   tccaacccgg taagacacga cttatcgcca ctggcagcag ccactggtaa caggattagc 2400
   agagcgaggt atgtaggcgg tgctacagag ttcttgaagt ggtggcctaa ctacggctac 2460
   actagaagaa cagtatttgg tatctgcgct ctgctgaagc cagttacctt cggaaaaaga 2520
   gttggtagct cttgatccgg caaacaaacc accgctggta gcggtggttt ttttgtttgc 2580
   aagcagcaga ttacgcgcag aaaaaaagga tctcaagaag atcctttgat cttttctacg 2640
   gggtctgacg ctcagtggaa cgaaaactca cgttaaggga ttttggtcat gagattatca 2700
   aaaaggatct tcacctagat ccttttaaat taaaaatgaa gttttaaatc aatctaaagt 2760
   atatatgagt aaacttggtc tgacagttac caatgcttaa tcagtgaggc acctatctca 2820
   gcgatctgtc tatttcgttc atccatagtt gcctgactcc ccgtcgtgta gataactacg 2880
   atacgggagg gcttaccatc tggccccagt gctgcaatga taccgcgaga cccacgctca 2940
   ccggctccag atttatcagc aataaaccag ccagccggaa gggccgagcg cagaagtggt 3000
   cctgcaactt tatccgcctc catccagtct attaattgtt gccgggaagc tagagtaagt 3060
   agttcgccag ttaatagttt gcgcaacgtt gttgccattg ctacaggcat cgtggtgtca 3120
   cgctcgtcgt ttggtatggc ttcattcagc tccggttccc aacgatcaag gcgagttaca 3180
   tgatccccca tgttgtgcaa aaaagcggtt agctccttcg gtcctccgat cgttgtcaga 3240
   agtaagttgg ccgcagtgtt atcactcatg gttatggcag cactgcataa ttctcttact 3300
   gtcatgccat ccgtaagatg cttttctgtg actggtgagt actcaaccaa gtcattctga 3360
   gaatagtgta tgcggcgacc gagttgctct tgcccggcgt caatacggga taataccgcg 3420
   ccacatagca gaactttaaa agtgctcatc attggaaaac gttcttcggg gcgaaaactc 3480
   tcaaggatct taccgctgtt gagatccagt tcgatgtaac ccactcgtgc acccaactga 3540
   tcttcagcat cttttacttt caccagcgtt tctgggtgag caaaaacagg aaggcaaaat 3600
   gccgcaaaaa agggaataag ggcgacacgg aaatgttgaa tactcatact cttccttttt 3660
   caatattatt gaagcattta tcagggttat tgtctcatga gcggatacat atttgaatgt 3720
   atttagaaaa ataaacaaat aggggttccg cgcacatttc cccgaaaagt gccacctgac 3780
   gtctaagaaa ccattattat catgacatta acctataaaa ataggcgtat cacgaggccc 3840
   tttcgtc 3847
<210> 3
   <211> 3177
   <212> DNA
   <213> Artificial
<220>
   <223> pA717N
<400> 3
   tcgcgcgttt cggtgatgac ggtgaaaacc tctgacacat gcagctcccg gagacggtca      60
   cagcttgtct gtaagcggat gccgggagca gacaagcccg tcagggcgcg tcagcgggtg     120
   ttggcgggtg tcggggctgg cttaactatg cggcatcaga gcagattgta ctgagagtgc     180
   accatatatg cggtgtgaaa taccgcacag atgcgtaagg agaaaatacc gcatcaggcg     240
   cgccattcgc cattcaggct gcgcaactgt tgggaagggc gatcggtgcg ggcctcttcg     300
   ctattacgcc agctggcgaa agggggatgt gctgcaaggc gattaagttg ggtaacgcca     360
   gggttttccc agtcacgacg ttgtaaaacg acggccagtg ccaagcttgc atgcctgcag     420
   aattcgagct cggtaccatg caggtggcgc gccgcggcct caccaacgcg attgtgagct     480
   atctcgagga caacgggtca gggcgacgtg agtaaccagt aaccgggttg tgacctccct     540
   tggtagcggc atttccgcta ccaacaagta ggctgttgca tatccccgtg tgtacgacca     600
   gcacggcata ctctagaagg agaagtacat atgaccggac tatcgcgacg caacgttctg     660
   atcggttcgc tcgtggcggc agctgccgtc ggtgcgggcg tcggtggcgc cgcaccggca     720
   ttcgccggcc cggggtcgac tctagctaga ttataaggat cctcgcgcaa cggttgccgc     780
   tactgggctt gacggcaaga cgccgtacag tagtgtgttc ggcaccttga acgctggtcc     840
   gccatgttca acgagccggt ctacctgccc gcaccgaaca agctggtgac atcgacccat     900
   gcggtgcggt accgagctcg aattaccggt aagcttctgc agaattcgta atcatgtcat     960
   agctgtttcc tgtgtgaaat tgttatccgc tcacaattcc acacaacata cgagccggaa    1020
   gcataaagtg taaagcctgg ggtgcctaat gagtgagcta actcacatta attgcgttgc    1080
   gctcactgcc cgctttccag tcgggaaacc tgtcgtgcca gctgcattaa tgaatcggcc    1140
   aacgcgcggg gagaggcggt ttgcgtattg ggcgctcttc cgcttcctcg ctcactgact    1200
   cgctgcgctc ggtcgttcgg ctgcggcgag cggtatcagc tcactcaaag gcggtaatac    1260
   ggttatccac agaatcaggg gataacgcag gaaagaacat gtgagcaaaa ggccagcaaa    1320
   aggccaggaa ccgtaaaaag gccgcgttgc tggcgttttt ccataggctc cgcccccctg    1380
   acgagcatca caaaaatcga cgctcaagtc agaggtggcg aaacccgaca ggactataaa    1440
   gataccaggc gtttccccct ggaagctccc tcgtgcgctc tcctgttccg accctgccgc    1500
   ttaccggata cctgtccgcc tttctccctt cgggaagcgt ggcgctttct catagctcac    1560
   gctgtaggta tctcagttcg gtgtaggtcg ttcgctccaa gctgggctgt gtgcacgaac    1620
   cccccgttca gcccgaccgc tgcgccttat ccggtaacta tcgtcttgag tccaacccgg    1680
   taagacacga cttatcgcca ctggcagcag ccactggtaa caggattagc agagcgaggt   1740
   atgtaggcgg tgctacagag ttcttgaagt ggtggcctaa ctacggctac actagaagaa   1800
   cagtatttgg tatctgcgct ctgctgaagc cagttacctt cggaaaaaga gttggtagct   1860
   cttgatccgg caaacaaacc accgctggta gcggtggttt ttttgtttgc aagcagcaga   1920
   ttacgcgcag aaaaaaagga tctcaagaag atcctttgat cttttctacg gggtctgacg   1980
   ctcagtggaa cgaaaactca cgttaaggga ttttggtcat gagattatca aaaaggatct   2040
   tcacctagat ccttttaaat taaaaatgaa gttttaaatc aatctaaagt atatatgagt   2100
   aaacttggtc tgacagttac caatgcttaa tcagtgaggc acctatctca gcgatctgtc   2160
   tatttcgttc atccatagtt gcctgactcc ccgtcgtgta gataactacg atacgggagg   2220
   gcttaccatc tggccccagt gctgcaatga taccgcgaga cccacgctca ccggctccag   2280
   atttatcagc aataaaccag ccagccggaa gggccgagcg cagaagtggt cctgcaactt   2340
   tatccgcctc catccagtct attaattgtt gccgggaagc tagagtaagt agttcgccag   2400
   ttaatagttt gcgcaacgtt gttgccattg ctacaggcat cgtggtgtca cgctcgtcgt   2460
   ttggtatggc ttcattcagc tccggttccc aacgatcaag gcgagttaca tgatccccca   2520
   tgttgtgcaa aaaagcggtt agctccttcg gtcctccgat cgttgtcaga agtaagttgg   2580
   ccgcagtgtt atcactcatg gttatggcag cactgcataa ttctcttact gtcatgccat   2640
   ccgtaagatg cttttctgtg actggtgagt actcaaccaa gtcattctga gaatagtgta   2700
   tgcggcgacc gagttgctct tgcccggcgt caatacggga taataccgcg ccacatagca   2760
   gaactttaaa agtgctcatc attggaaaac gttcttcggg gcgaaaactc tcaaggatct   2820
   taccgctgtt gagatccagt tcgatgtaac ccactcgtgc acccaactga tcttcagcat   2880
   cttttacttt caccagcgtt tctgggtgag caaaaacagg aaggcaaaat gccgcaaaaa   2940
   agggaataag ggcgacacgg aaatgttgaa tactcatact cttccttttt caatattatt   3000
   gaagcattta tcagggttat tgtctcatga gcggatacat atttgaatgt atttagaaaa   3060
   ataaacaaat aggggttccg cgcacatttc cccgaaaagt gccacctgac gtctaagaaa   3120
   ccattattat catgacatta acctataaaa ataggcgtat cacgaggccc tttcgtc      3177
<210> 4
   <211> 4407
   <212> DNA
   <213> Artificial
<220>
   <223> pSO246
<400> 4
   ggcctctaac cgctcgacgc gctgcaccaa ccagcccgcg aacggctggc agccagcgta 60
   aggcgcggct catcgggcgg cgttcgccac gatgtcctgc acttcgagcc aagcctcgaa 120
   cacctgctgg tgtgcacgac tcacccggtt gttgacaccg cgcgcggccg tgcgggctcg 180
   gtggggcggc tctgtcgccc ttgccagcgt gagtagcgcg tacctcacct cgcccaacag 240
   gtcgcacaca gccgattcgt acgccataaa gccaggtgag cccaccagct ccgtaagttc 300
   gggcgctgtg tggctcgtac ccgcgcattc aggcggcagg gggtctaacg ggtctaaggc 360
   ggcgtgtacg gccgccacag cggctctcag cggcccggaa acgtcctcga aacgacgcat 420
   gtgttcctcc tggttggtac aggtggttgg gggtgctcgg ctgtcgctgg tgttccacca 480
   ccagggctcg acgggagagc gggggagtgt gcagttgtgg ggtggcccct cagcgaaata 540
   tctgacttgg agctcgtgtc cggaccatac accggtgatt aatcagtggt ctactaccaa 600
   gcgtgagcca cgtccgccga cgaatttgaa gcagctctgg ctgccgtact ggccgctggc 660
   aagcgacgat ctgctcgagg ggatctaccg ccaaagccgc gcgtcggccc taggccgccg 720
   gtacatcgag gcgaacccaa cagcgctggc aaacctgctg gtcgtggacg tagaccatcc 780
   agacgcagcg ctccgagcgc tcagcgcccg ggggtcccat ccgctgccca acgcgatcgt 840
   gggcaatcgc gccaacggcc acgcacacgc agtgtgggca ctcaacgccc ctgttccacg 900
   caccgaatac gcgcggcgta agccgctcgc atacatggcg gcgtgcgccg aaggccttcg 960
   gcgcgccgtc gacggcgacc gcagttactc aggcctcatg accaaaaacc ccggccacat 1020
   cgcctgggaa acggaatggc tccactcaga tctctacaca ctcagccaca tcgaggccga 1080
   gctcggcgcg aacatgccac cgccgcgctg gcgtcagcag accacgtaca aagcggctcc 1140
   gacgccgcta gggcggaatt gcgcactgtt cgattccgtc aggttgtggg cctatcgtcc 1200
   cgccctcatg cggatctacc tgccgacccg gaacgtggac ggactcggcc gcgcgatcta 1260
   tgccgagtgc cacgcgcgaa acgccgaatt cccgtgcaac gacgtgtgtc ccggaccgct 1320
   accggacagc gaggtccgcg ccatcgccaa cagcatttgg cgttggatca caaccaagtc 1380
   gcgcatttgg gcggacggga tcgtggtcta cgaggccaca ctcagtgcgc gccagtcggc 1440
   catctcgcgg aagggcgcag cagcgcgcac ggcggcgagc acagttgcgc ggcgcgcaaa 1500
   gtccgcgtca gccatggagg cattgctatg agcgacggct acagcgacgg ctacagcgac 1560
   ggctacaacc ggcagccgac tgtccgcaaa aagcggcgcg tgaccgccgc cgaaggcgct 1620
   cgaatcaccg gactatccga acgccacgtc gtccggctcg tggcgcagga acgcagcgag 1680
   tggctcgccg agcaggctgc acgccgcgaa cgcatccgcg cctatcacga cgacgagggc 1740
   cactcttggc cgcaaacggc caaacatttc gggctgcatc tggacaccgt taagcgactc 1800
   ggctatcggg cgaggaaaga gcgtgcggca gaacaggaag cggctcaaaa ggcccacaac 1860
   gaagccgaca atccaccgct gttctaacgc aattggggag cgggtgtcgc gggggttccg 1920
   tggggggttc cgttgcaacg ggtcggacag gtaaaagtcc tggtagacgc tagttttctg 1980
   gtttgggcca tgcctgtctc gttgcgtgtt tcgttgcgcc cgttttgaat accagccaga 2040
   cgagacgggg ttctacgaat cttggtcgat accaagccat ttccgctgaa tatcggggag 2100
   ctcaccgcca gaatcggtgg ttgtggtgat gtacgtggcg aactccgttg tagtgcctgt 2160
   ggtggcatcc gtggccactc tcgttgcacg gttcgttgtg ccgttacagg ccccgttgac 2220
   agctcaccga acgtagttaa aacatgctgg tcaaactagg tttaccaacg atacgagtca 2280
   gctcatctag ggccagttct aggcgttgtt cgttgcgcgg ttcgttgcgc atgtttcgtg 2340
   tggttgctag atggctccgc aaccacacgc ttcgaggttg agtgcttcca gcacgggcgc 2400
   gatccagaag aacttcgtcg tgcgactgtc ctcgttctag agtcgaccaa agcggccatc 2460
   gtgcctcccc actcctgcag ttcgggggca tggatgcgcg gatagccgct gctggtttcc 2520
   tggatgccga cggatttgca ctgccggtag aactccgcga ggtcgtccag cctcaggcag 2580
   cagctgaacc aactcgcgag gggatcgagc ccggggtggg cgaagaactc cagcatgaga 2640
   tccccgcgct ggaggatcat ccagccggcg tcccggaaaa cgattccgaa gcccaacctt 2700
   tcatagaagg cggcggtgga atcgaaatct cgtgatggca ggttgggcgt cgcttggtcg 2760
   gtcatttcga accccagagt cccgctcaga agaactcgtc aagaaggcga tagaaggcga 2820
   tgcgctgcga atcgggagcg gcgataccgt aaagcacgag gaagcggtca gcccattcgc 2880
   cgccaagctc ttcagcaata tcacgggtag ccaacgctat gtcctgatag cggtccgcca 2940
   cacccagccg gccacagtcg atgaatccag aaaagcggcc attttccacc atgatattcg 3000
   gcaagcaggc atcgccatgg gtcacgacga gatcctcgcc gtcgggcatg cgcgccttga 3060
   gcctggcgaa cagttcggct ggcgcgagcc cctgatgctc ttcgtccaga tcatcctgat 3120
   cgacaagacc ggcttccatc cgagtacgtg ctcgctcgat gcgatgtttc gcttggtggt 3180
   cgaatgggca ggtagccgga tcaagcgtat gcagccgccg cattgcatca gccatgatgg 3240
   atactttctc ggcaggagca aggtgagatg acaggagatc ctgccccggc acttcgccca 3300
   atagcagcca gtcccttccc gcttcagtga caacgtcgag cacagctgcg caaggaacgc 3360
   ccgtcgtggc cagccacgat agccgcgctg cctcgtcctg cagttcattc agggcaccgg 3420
   acaggtcggt cttgacaaaa agaaccgggc gcccctgcgc tgacagccgg aacacggcgg 3480
   catcagagca gccgattgtc tgttgtgccc agtcatagcc gaatagcctc tccacccaag 3540
   cggccggaga acctgcgtgc aatccatctt gttcaatcat gcgaaacgat cctcatcctg 3600
   caaaaggcca gcaaaaggcc aggaaccgta aaaaggccgc gttgctggcg tttttccata 3660
   ggctccgccc ccctgacgag catcacaaaa atcgacgctc aagtcagagg tggcgaaacc 3720
   cgacaggact ataaagatac caggcgtttc cccctggaag ctccctcgtg cgctctcctg 3780
   ttccgaccct gccgcttacc ggatacctgt ccgcctttct cccttcggga agcgtggcgc 3840
   tttctcatag ctcacgctgt aggtatctca gttcggtgta ggtcgttcgc tccaagctgg   3900
   gctgtgtgca cgaacccccc gttcagcccg accgctgcgc cttatccggt aactatcgtc   3960
   ttgagtccaa cccggtaaga cacgacttat cgccactggc agcagccact ggtaacagga   4020
   ttagcagagc gaggtatgta ggcggtgcta cagagttctt gaagtggtgg cctaactacg   4080
   gctacactag aagaacagta tttggtatct gcgctctgct gaagccagtt accttcggaa   4140
   aaagagttgg tagctcttga tccggcaaac aaaccaccgc tggtagcggt ggtttttttg   4200
   tttgcaagca gcagattacg cgcagaaaaa aaggatctca agaagatcct ttgatctttt   4260
   ctacggggtc tgacgctcag tggaacgaaa actcacgtta agggatggta ccgggccccc   4320
   cctcgaggtc gacggtatcg ataagcttga tatcgaattc ctgcagcccg ggggatcctg   4380
   tagatcgaac cgaaaacgtg tcgaagt                                       4407